# EUROPEAN PATENT SPECIFICATION

(11) **EP 1 655 036 B1**
(45) Date of publication and mention of the grant of the patent: **17.07.2013**
(21) Application number: 04748955.4
(22) Date of filing: 01.07.2004
(51) Int. Cl.: A61K 38/46, A61P 35/00

(54) **METHOD FOR TREATING ONCOLOGICAL DISEASES**
VERFAHREN ZUR BEHANDLUNG VON ONKOLOGISCHEN ERKRANKUNGEN
METHODE DE TRAITEMENT DE MALADIES ONCOLOGIQUES

(30) Priority: 14.07.2003 WO PCT/RU03/00304; 12.03.2004 RU 2004108061
(43) Date of publication of application: 10.05.2006
(73) Proprietor: CLS Therapeutics Limited, Guernsey GY1 3DR (GB)
(72) Inventor: GENKIN, Dmitry Dmitrievich, St.Petersburg, 197110 (RU); TETS, Viktor Veniaminovich, St.Petersburg, 196066 (RU); TETS, Georgy Viktorovich, Saint-Petersburg, 191040 (RU)
(74) Representative: Schneider, Henry
(86) International application number: PCT/RU2004/000261
(87) International publication number: WO 2005/004903

(56) References cited:
- RU-C1- 2 099 080
- RU-C2- 2 227 029
- US-A- 5 484 589
- US-B1- 6 521 409
- SUGIHARA S ET AL: "Deoxyribonuclease treatment prevents blood-borne liver metastasis of cutaneously transplanted tumour cells in mice" BRITISH JOURNAL OF CANCER, NATURE PUBLISHING GROUP, LONDON, GB, vol. 67, no. 1, 1 January 1993 (1993-01-01), pages 66-70, XP008086562 ISSN: 0007-0920
- MUTIRANGURA A.: 'Serum/plasma viral DNA: mechanism and diagnostic applications to nasopharyngeal and cervical carcinoma' ANN. N. Y. ACAD. SCI. vol. 945, September 2001, pages 59 - 67, XP008043289
- EL HASSAN N.O. ET AL.: 'Rescue use of Dnase in critical lung atelectas mucus retention in premature neonates' PEDIATRICS vol. 108, no. 2, August 2001, pages 468 - 470, XP002984504

## Description

### Technical field

The invention is related to medicine and veterinary and can be used for treating in the main of solid tumors. It refers to the use of recombinant human DNAse in treating a patient having cancer.

### Background art

Populations of tumor cells, which develop in the body of the patient, have vert' high genetic variability, which exceeds the same of healthy cells. Genetic variability of cancer cell populations allows their generate phenotypes, which are insensitive for immune and morphogenetic control and which have ability to invasion and metastasis and are insensitive for cancer therapy. It is considered that selection and clonal expansion of cancer cells underlie biological and clinical «progression» of tumors. That's why the strategy of modem cancer therapy is based on the principle of destroying of cancer cells clones in the body of patient with chemotherapy, radiotherapy, immunotherapy, biotherapy, surgical methods and the combination thereof.

Chemotherapy, radiotherapy, biotherapy and more recently the immunotherapy are the most-used non-surgical methods of treating of cancer diseases and its arc meant for destruction, damage or inactivation of cancer cell intracellular DNA.

The chemotherapy approach is based on well known compounds: for platinum preparations see "Molecular mechanisms involved in cisplatin cytotoxicity". Jordan P, Carmo-Fonseca M, Cell Mol Life Sci 2000 Aug. v 57: pp.1229-35, for anthracycline antibiotics, see "Daunorubicin and doxorubicin, anthracycline antibiotics, a physicochemical and biological review", Aubel-Sadron G, Londos-Gagliardi D, Biochimie 1984 May v.66: pp. 333-52, for alkylating agents, see "An overview of cyclophosphamide and ifosfamide pharmacology" and for podophyllotoxins, see "Podophyllotoxins: current status and recent developments", Damayanthi Y, Lown JW, Curr Med Chem 1998 Jun 5: v 3 205-52.

Methods of radioimmunotherapy which allow to irradiate the intracellular DNA of cancer cells' nuclei by alpha particles from alpha-emitters which are specially delivered into cancer cells for increasing the effect on intracellular DNA, obtain the recognition, see "Targeted alpha therapy: evidence for potential efficacy of alpha-immunoconjugates in the management of micrometastatic cancer" Allen BJ, Australas Radiol 1999 Nov v.43: pp 480-6..

There are biotherapeutic and immunotherapeutic methods, which meant for induction of apoptosis of cancer cells - the process of death of cancer cell, which starts with the activation of intracellular nucleases with following degradation of intracellular DNA of the tumor cell, for example by means administration of genotherapeutic constructions, which consists of genes inducing apoptosis, see "Phase I trial of adenovirus-mediated p53 gene therapy for recurrent glioma: biological and clinical results" Lang FF, Bruner JM, Fuller GN, Aldape K, Prados MD, Chang S, Berger MS, McDermott MW, Kunwar SM, Junck LR, Chandler W, Zwiebel JA, Kaplan RS, Yung WK, J Clin Oncol 2003 Jul 1 21:13 2508-18, or genes coding the factors which activate the nucleases triggering apoptosis, see "Adenovirus-mediated transfer of caspase-8 augments cell death in gliomas: implication for gene therapy." Shinoura N, Koike H, Furitu T, Hashimoto M, Asai A, Kirino T, Hamada H, Hum Gene Ther 2000 May 20 11:8 1123-37, or by meant of anticancer vaccines, see "Vaccine-induced apoptosis: a novel clinical trial end point?" Amin S, Robins RA, Maxwell-Armstrong CA, Scholefield JH, Durrant LG,Cancer Res 2000 Jun 60: 3132-6.

Use of endonuclease Endo SR for treatment cancer diseases by moode of intracellular delivery it into target cells is known, US, C, 6455250.

From methods listed above, the chemotherapy with the Etopozide -4-Demetilpipodophylotoxe (4,6-O-R)- etiliden)-b-D-glycopiranozid was selected by us as a prototype.

Topoizomeraze II is essential cell enzyme which regulates many aspects of DNA function. The enzyme is responsible for interconversion of different topological forms of intracellular DNA by means of generation of transitory breaks of double-stranded DNA. Etopozide, as Topoizomeraze II inhibitor, increases intracellular level of "broken DNA - Topoizomeraze II" complexes.

The result of this drug influence is the accumulation of the double-stranded intracellular DNA breaks which lead to cell death, see "Topoisomerase II as a target for anticancer drugs: when enzymes stop being nice". Fortune JM, Osheroff N., Prog Nucleic Acid Res Mol Biol 2000, v. 64: pp.221-53.

The drawback of the method-prototype as well as any other well-known methods, is their low efficacy. It is explained by following. Method-prototype as well as other known methods implies the cancer cell and mostly the intracellular DNA as a therapeutic target.

The experience of such therapy testifies that:
- because of high genetic variability, cancer cells become, as a rule no sensitive to the therapy before they are adequately eliminated by means of the using method;
- intracellular DNA is a difficult-to-approach target, and it leads to necessity of high-dosing antineoplastic chemotherapy and/or necessity of complicated delivery systems (Drug Delivery Systems). As well, method-prototype which makes provision for influence on intracellular DNA of cancer cells also damages DNA of healthy cells and that's reason of it's high toxicity.

Sugihara S. et al. disclose that systemic introduction of bovine pancreatic DNAse I (0.1 Kunitz units (KU) per mouse per day) could slow down liver metastases development. The authors describe that an effect of DNase alone is not satisfactory and requires a combination with surgical removal of the primary tumor (Sugihara S. et al. British Journal of Cancer. Nature Publishing Group, London, GB, vol. 67, No. 1, 1 January 1993, pages 66-70; XP008086562ISSN: 0007-0920).

### Disclosure of the invention

The solving of the aim of development of highly efficient and low-toxic method of cancer therapy is the basis of this invention. According to the invention this problem is resolved by administrating into systemic circulation an agent which destroys blood extracellular DNA; and the said agent can be introduced in the dose which provide the alteration an electrophoretic profile of blood extracellular DNA, which could be detectable by puls-gelelectrophoresis; the agent can be introduced in doses and regime schedule providing a plasma hydrolytic activity, measured in blood plasma which exceeds 150 Kunitz units/liter of blood plasma, and this level can be supported for more than 12 hours within 24 hours in total; the treatment can be carried out continuously no less than 48 hours; DNAase enzyme can be used as agent to destroy extracellular blood DNA; whereby as agent recombinant human DNAase I is parenterally introduced in doses ranged from 0.15 mg/day till 500 mg/day per day every day during period of 5-360 days. The treatment can be life long.

The fact of circulation of extracellular DNA in blood of cancer patients was described in a number of papers, see (P.Anker et al., Clinica Chimica Acta, v.313, 2001, pp.143-146; Fedorov N.A. et.al., Bull.Exp.Biol.Med.,v102,1986, pp. 281-283). In patent US,C,5952170 the determination of extracellular DNA in blood for diagnostics and prognosis of clinical course of a malignant disease was described. In patents US, C, 6465177 and US, C, 6156504 there was described the use of extracellular DNA of blood for definition of mutations in oncogenes and microsatellic fragments of genes. Usage of blood extracellular DNA for studying the genome instability in tumors and application of the results for diagnostics, monitoring and prognosing were also disclosed.

Besides, there was no systematic analysis of extracellular blood DNA spectrum and it's biological role before now. Published data of investigation of blood extracellular DNA performed without polymerase chain reaction (PCR) method were not found. PCR can pervert the pattern of blood extracellular DNA because of specificity of primers which are used for amplification. Until recent times the genetic analysis of extracellular blood DNA was mainly carried out by PCR or blothybridization and it was directed to the studying of changes in certain fragments of genome, for example in micro satellites and separate genes during malignant process (Sanchez-Cespedes M., et al., Ann Oncol, 1998, v9(1), pp.113-116; Sozzi G., et al., Clin Can Res, 1999, v. 5(10), pp.2689-2692; Chen X.Q., et al., Nat Med, 1996, v. 2(9), pp.1033-1035).

Thus, in source of available data, there are no knowledge about genetic repertoire of extracellular blood DNA of cancer patients, biological role of extracellular blood DNA in oncopatology and potential therapeutic effects of destroying or inactivation for treating these diseases and according to aforesaid the invention conformances to requirements of "novelty" criteria (N).

As the applicant established, the extracellular blood DNA of cancer patients contains the unique quantitative and qualitative repertoire of genes and regulating genetic elements which great differ from the repertoire of DNA which is described in human genome. In contrast to inracellular DNA the extracellular DNA of cancer patients contains mainly unique human genes, including genes which are involved in development and maintenance of malignant behavior of cancer cells.

It is shown that extracellular DNA of blood of cancer patients contributes the malignant growth.

Destroying, modification and binding of extracellular blood DNA slows down the malignant growth. Such intervention is very useful as independent therapy and it also increases the effectiveness of traditional methods of treatment.

Aforesaid new characteristics of this invention are based on new ideas about mechanisms of oncology diseases. In this way this method conformances to requirements of "invention step" criteria (IS).

### Brief description of the drawings

As set for below the invention has been explained by detailed description of embodiments without references to drawings.

### Preferred embodiment

The inventive method is realized as followed:

### Materials and methods.

The following agents were used which destroys extracellular blood DNA: bovine pancreatic DNAase (Sigma and Samson Med), recombinative human DNAase 1 (Dornase alpha; Genetech), Serratia Mercenses extracellular nuclease. The solutions of DNA-se for administration was made by dissolving of mother solutions of DNA-se in sterile phosphate buffer just before administration.

Extracellular DNA from blood plasma was isolated as follows: fresh plasma (no more than 3-4 hours after sampling) was centrifuged on Ficoll-PlaquePlus (Amersham-Pharmacia) during 20 minutes at 1500 g at room temperature. ½ of plasma was detached, not affecting the rest of cells on the Ficoll pillow, and further centrifuged at 10000 g during 30 min for separation from cell fragments and debris. Supernatant was detached, without affecting of the sediment, and was toped up to 1% of sarkosil ,50MM tris-HCl, pH 7,6, 20 MM EDTA, 400 MM NaCl, and than mixed with equal volume of phenol-chloroform(1:1) mixture. The prepared emulsion was incubated during 2 hours at t=65°C, then phenol-chloroform mixture was separated by centrifuging (500g during 20 minutes, room temperature).

The procedure of deproteinisation with phenol - chlorophorm mixture was repeated 3 times, and then the water phase was processed with chloroform and diethyl ether. Separation from organic solvents was made by centrifugation at 5000g during 15 minutes). Then equal volume of izopropanol was added to resulting aqueous phase and the mixture was incubated overnight at 0°C. After sedimentation the nucleic acids were separated by centrifugation at 10000g during 30 minutes. The sediment of nucleic acids was dissolved in of 10MM tris-HCl buffer, pH 7, 6 with 5 MM EDTA, and inflicted to the CsCl gradient (1M, 2.5M, 5.7M) in test-tube for rotor SW60Ti. The volume of DNA solution was 2 ml, volume of each step of CsCl was 1 ml. Ultracentrifugation was conducted in L80-80 (Beckman) centrifuge during 3 hours at 250000g. DNA was collected from the surface of each gradient step into fractions. These fractions were dialyzed during 12 hours (t=4°C). Presence of DNA in fractions was determined by agar electrophoresis and DNA was visualized by ethidium bromide staining. The amount of DNA was determined with specrophotometer (Beckman DU70) in cuvet (100 mcl) at wavelength of 220-230 nm.

Mice Lewis lung carcinoma and Erlich carcinoma were used in experiments. Cells were cultivated in RPMI-1640 medium with 10% calf serum and 1% penicillin-streptomycin in atmosphere of 5% CO2.

For tumors inoculation in mice the cells were cultivated till monolayer is formed, then detached with tripsin-EDTA buffer. The cells were washed 3 times by centrifuging in phosphate buffer and then resuspended up to 0, 5*10⁷ /ml concentration in the same buffer. The cell viability was determined with metylene blue staining in haemocitometer. Cells suspensions with no less than 95% of living cell were used for transplantation.

C57B1 mice and white randomly breeded mice from "Rappolovo" animal house were used. Weight of animals was 24-26 g. 6-7animals were kept in one cage on a standard diet without limitation of water. LLC cells in dose 5*10⁵ per mice in 0,1ml of phosphate buffer were transplanted into thigh soft tissues. Erlich tumors were transplanted by administration of 0,2 ml of 10% cell suspension in physiological solution.

In some experiments level of extracellular DNA in blood plasma was quantified. DNA was isolated according to aforesaid protocol. The DNA level was measured with PicoGreen kit. Electrophoresis of extracellular blood DNA was performed with 1% agar gel. DNA was visualized with etidium-bromide solution. The levels of high molecular DNA fraction (more than 300 base pairs) were determined by densitometry. Lambda phage DNA, digested with EcoRI and HindIII was used as electrophoresis marker.

### Example 1. Iinhibition of Erlich carcinoma growth.

Recombinant human DNAase 1 (Genentech) was used.
1 group: 10 mice bearing Erlich carcinoma was used as control. The mice were injected with 0,2 ml of phosphate buffer intraperitoneally twice a day every day from day 3 to day 7 after the tumor cell transplantation.
2 group: 10 mice bearing Erlich carcinoma were introduced with intraperitoneal injections of DNAase in dose of 1 mg/kg of body weight in 0,2ml of phosphate buffer four times daily every day from day 3 to day 7 after the tumor cell transplantation.
3 group: 10 mice bearing Erlich carcinoma were administered with intraperitoneal injections of DNAase in dose of 0,5 mg/kg of body weight in 0,2ml of phosphate buffer four times daily every day from day 3 to day 7 after the tumor cell transplantation.
4 group: 10 mice bearing Erlich carcinoma were administered with intraperitoneal injections of DNAase in dose of 0,1 mg/kg of body weight in 0,2ml of phosphate buffer four times daily every day from day 3 to day 7 after the tumor cell transplantation.
5 group: 10 mice bearing Erlich carcinoma were administered with intraperitoneal injections of DNAase in dose of 0,05 mg/kg of body weight in 0,2ml of phosphate buffer four times daily every day from day 3 to day 7 after the tumor cell transplantation. The results were evaluated as tumor Growth Inhibitory Index(GII) (%) at the last day of DNAase injections. The quantification of blood plasma extracellular DNA and it's electrophoretic qualification were also performed.

The results are presented in the table 1.

Tumor size, extracellular DNA level and extracellular DNA electrophoresis profile on day 7 after tumor transplantation.

**Table 1**

| Group | Tumor volume | Inhibition (%) | Extracellular DNA level, (ng/ml) | Presence of high molecular fractions of extracellular DNA |
|---|---|---|---|---|
| Control | 98+/-14 | - | 104,8 | 100%* |
| 1mg/kg\ | 23+/-6 | 76% | 38,3 | 0 |
| 0,5mg/kg | 32+/-6 | 67% | 55,1 | 25% |
| 0,1mg/kg | 58+/-12 | 37% | 78,0 | 70% |
| 0,05 mg/kg | 87+/-11 | 10% | 98,7 | 100% |

| | | | | |
|---|---|---|---|---|
| * The control group electrophoretic density were considered as 100%. | | | | |

The presented data demonstrated that sufficiently high doses of DNAase 1 are needed to achieve the better therapeutic effect.

### Example 2. Iinhibition of Erlich carcinoma growth.

Recombinant human DNAase I (Genentech) was used.
5 groups of mice bearing LLC were used.
1 group -7 mice - the control.
2 group - 6 mice were administered with intraperitoneal injections of DNAase in dose of 1 mg/kg of body weight twice a day every day from day 3 to day 5 after the tumor cell transplantation.
3 group - 6 mice were administered with intraperitoneal injections of DNAase in dose of 1 mg/kg of body weight twice a day every day from day 3 to day 10 after the tumor cell transplantation.
4 group - 6 mice were administered with intraperitoneal injections of DNAase in dose of 1 mg/kg of body weight twice a day every day from day 3 to day 15 after the tumor cell transplantation.
5 group - 6 mice were administered with intraperitoneal injections of DNAase in dose of 1 mg/kg of body weight twice a day every day from day 3 to day 18 after the tumor cell transplantation.
6 group - 6 mice were administered with intraperitoneal injections of DNAase in dose of 1 mg/kg of body weight twice a day every day on 3,5,7,9,11,13,15 and 17 day after the tumor cell transplantation.
7 group - 6 mice were administered with intraperitoneal injections of DNAase in dose of 0,5 mg/kg of body weight four times daily every day from day 3 to day 10 after the tumor cell transplantation. The results was evaluated as animal survival on day 30 and day 50 after the tumor cell transplantation. The results are presented in the table 2.

Animal survival on day 30 and day 50 after the tumor cell transplantation.

**Table 2**

| Group | day 30 (amount of alive/ dead animals in group | day 50 (amount of alive/ dead animals in group) |
|---|---|---|
| 1 | 0-7 | 0-7 |
| 2 | 0-6 | 0-6 |
| 3 | 3-3 | 0-6 |
| 4 | 5-1 | 3-3 |
| 5 | 6-0 | 6-0 |
| 6 | 0-6 | 0-6 |
| 7 | 4-2 | 1-5 |

The presented data demonstrated that the therapy efficacy increases as the treatment time extends. The therapy efficacy is decrease if it is not uninterrupted. Multiple-dose administration is preferred.

### Example. 3. Lung carcinoma treatment.

54 -years -old man has been admitted to the hospital with diagnosis of lung carcinoma.

By patient's agreement, due to lack of any available treatment modality, subcutaneous injections of dornaze - alpha were prescribed. The treatment began with administration of daily dose of 50 mkg/kg. Every consecutive day blood extracellular DNA level was measured and blood extracellular DNA was fractioned by electrophoresis. Once a week the primary tumor site and metastases were checked with X-rays and NMR-tomography. After initial 7 day period the domaze-alpha daily dose has been increased up to 100 mkg/kg because of no changes in level and electrophoresis pattern of blood extracellular DNA and no reactions from primary site of the tumor and the metastases. Because of no changes after another 7 days the dosing has been increased up to 150 mkg/kg. Two days after the first injection of the preparation in dose 150 mkg/kg the material recession (more than 50%) of the blood extracellular DNA fraction with the size more than 300 base pairs has been observed although total amount of extracellular DNA has not been greatly decreased (less than 20%). During the next 4 days the patient's general condition has noticeably improved and on day 7 of this cycle of therapy 25% - decreasing of primary tumor lesion size and signs of regression of two bone metastases have been shown by NMR-scanning and X-ray examination. The probes of patient's extracellular DNA taken before the treatment started and 21 days after the beginning the therapy were cloned by means a method which allowed to construct non amplificated plasmid libraries of blood extracellular DNA with representativeness up to one million of clones with the average size of 300-500 base pairs. The DNA which have been isolated with aforesaid protocol was additionally deproteinizated with proteinase K (Sigma) at t=65 C for the removing of firm-binded proteins. After the deproteinization and single-stage treatment of phenol-chloroform mixture (t= 65°C) DNA was precipitated overnight with 2,5 volumes of ethanol. Then DNA was treated by Eco RI restrictase during 3 hours or by Pfu polymerase (Stratagene) in presence of 300 mkM of all desoxynucleotidtriphosphates for sticky-ends elimination. The completed DNA was phosphorylated by polynucleotidkinase T4 (30U, 2 h.). The preparations were ligated to pBluescript plasmide (Stratagene), which had been digested with EcoRI or PvuII and dephosphorylated by phosphatase CIP (Fermentas) during 1 hour. 1 mkg of vector and 0,1-0,5 mkg of serum DNA were used. The process of ligation was conducted with Rapid Legation Kit (Roche) during 10 hours at T=16 °C. The volume of this mixture was 50 mkl. The ligated library was transformed into DH12S cells (Life Technologies) by meant of electroporator E. Coli porator (BioRad). 12-20 electroporation covets were used for the transformation of one library. The library serial dilutions of 10⁻⁴, 10⁻⁵ and 10⁻⁶ were cloned on 1,5% agar and LB media supplemented with 100 mkg\ml of ampicilline. In both cases the libraries represented 2-3*10⁶ clones.

Analysis of 96 randomly selected clones with the size 300-1000 base pairs from the "before treatment" library showed that 55 from 96 clones were the unique sequences of human DNA. For the 15 sequences from 55 the gene function or corresponding gene product were identified with HumanGeneBank.

| | |
|---|---|
| Gene or corresponding protein product | Reported role in cancerogenesis and cancer progression |
| G-protein coupled receptor protein | Key role in neoplastic transformation,apoptosis inhibition, hormone independence and metastasis |
| Snf2 coupled CBP activator (SCARP) | Transcription activator, reported in synovial sarcoma and leukemia. |
| SRY-box containing gene | Transcription modulator expressed in embryogenesis. Reported in medulloblastoma, gonadal tumors, highly metastatic melanoma. |
| Tyrosine kinase | Key role in cancer cell regulation network. Some class homologues are the products of cellular oncogenes. |
| Fibroblast activation protein, cell surface protease | Involved into cancer invasion and metastasis. |
| Brain testican | Reported in embryonic rhabdomyosarcoma. |
| KRAB domain, Zn-finger protein. | Reported in early embryogenesis, neuroblastoma, Ewing sarcoma, T-cell lymphoma, linked with acquisition of drug resistance in lung cancer. |
| Melanoma associated antigen | Antigen expressed in melanoma cells. |
| N-cadherin | Involved into cancer invasion and metastasis. |
| Interleukin 7 | Proposed essential autocrine - paracrine growth factor for many cancers |
| DEAD Box RNA helicase-like protein | Expressed in highly proliferating and cancer cells. |
| Lipin-1 | Involved into cancer cell response to cytotoxic drugs. |
| Dynein | Participate in p53 intracellular traffic, reported in prostate cancer and hepatocellular carcinoma. |
| Ramp protein | Reported in human embryonic carcinoma |

Analysis of 100 clones selected randomly from the "21 day after treatment" library showed that more than 90% sequences of clones represented short fragments of repetitive DNA of human genome with dominance of alpha-satellite DNA.

Hence the use of DNAase in doses which are sufficient for destroying extracellular blood DNA with size higher than 300 base pairs leads to disappearing of unique fragments of human genome from extracellular blood DNA, including those involved into development and maintenance of cancer cells malignant behavior. At the same time the tumor respond to applied therapy.

### Industrial applicability

For the realization the methods there were used well-known materials and equipment manufactured in plant conditions and according to aforesaid the invention conformances to requirements of "industrial applicability" criteria (IA).

## Claims

1. Recombinant human DNAse I for use in treating a patient having cancer, wherein said DNAse is to be parenterally administered into systemic blood circulation daily at 0.15 - 500 mg/kg during period of 5 - 360 days.

## Patentansprüche

1. Rekombinante menschliche DNAse I zur Verwendung bei der Behandlung eines Patienten mit Krebs, wobei die genannte DNAse parenteral in den systemischen Blutkreislauf mit einer täglichen Dosis von 0,15 - 500 mg / kg während eines Zeitraums von 5 - 360 Tagen verabreicht wird.

## Revendications

1. DNase I humaine recombinante pour l'utilisation dans le traitement d'un patient atteint d'un cancer , dans laquelle ladite DNase est destinée à être administrée par voie parentérale dans la circulation sanguine systémique quotidienne à 0,15 à 500 mg / kg pendant une période de 5 - 360 jours.
